(19) Europäisches Patentamt
European Patent Office
Office européen des brevets

(11) Publication number: 0 041 737
B1

(12) **EUROPEAN PATENT SPECIFICATION**

(45) Date of publication of patent specification: **29.08.84**

(21) Application number: **81104467.6**

(22) Date of filing: **10.06.81**

(51) Int. Cl.³: **C 07 C 143/70,**
C 07 C 69/708, C 07 F 9/40,
C 08 F 16/14, C 08 F 16/26,
C 08 F 16/30, C 08 F 216/14,
C 07 C 43/17, C 07 C 41/24

(54) **Process to produce novel fluorocarbon vinyl ethers and resulting polymers.**

(30) Priority: **11.06.80 US 158532**

(43) Date of publication of application:
**16.12.81 Bulletin 81/50**

(45) Publication of the grant of the patent:
**29.08.84 Bulletin 84/35**

(84) Designated Contracting States:
**AT BE CH DE FR GB IT LI NL SE**

(56) References cited:
**FR-A-1 341 087**
**FR-A-1 410 444**
**FR-A-1 422 147**
**GB-A-1 550 874**
**US-A-3 282 875**
**US-A-3 351 619**

(73) Proprietor: **THE DOW CHEMICAL COMPANY**
**Dow Center 2030 Abbott Road Post Office Box 1967**
**Midland Michigan 48640 (US)**

(72) Inventor: **Ezzell, Bobby Ray**
**5 Deer Court Lake Jackson**
**Texas 77566 (US)**
Inventor: **Carl, William Paul**
**P. O. Box 843 Angleton T**
**Texas 77515 (US)**
Inventor: **Mod, William August**
**214 Oyster Creek Drive Lake Jackson**
**Texas 77566 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

**EP 0 041 737 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel compounds prepared by decarboxylation of carboxylic acid derivatives.

U.S. 3,282,875 shows the following decarboxylation reaction

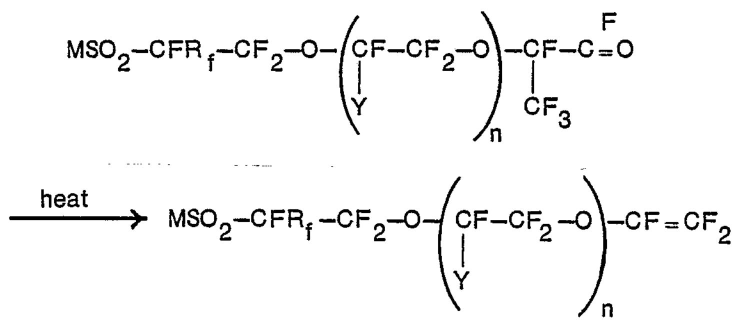


where

$R_f$ is F or a perfluoromethyl radical having from 1—10 carbon atoms;
y is F or trifluoromethyl radical;
n is an integer of 1—3, inclusive;
M is F, hydroxyl radical, amino radical or OMe; and
Me is an alkali metal or a quaternary nitrogen radical.

Yields in the decarboxylation reaction of about 80% were obtained at high temperatures (about 300°C) while yields of 20—30% were obtained at lower temperatures (about 200°C). Also taught is the homo and copolymerization of the vinylether monomers to form useful polymers.

British patent 1,518,387 teaches the following reactions

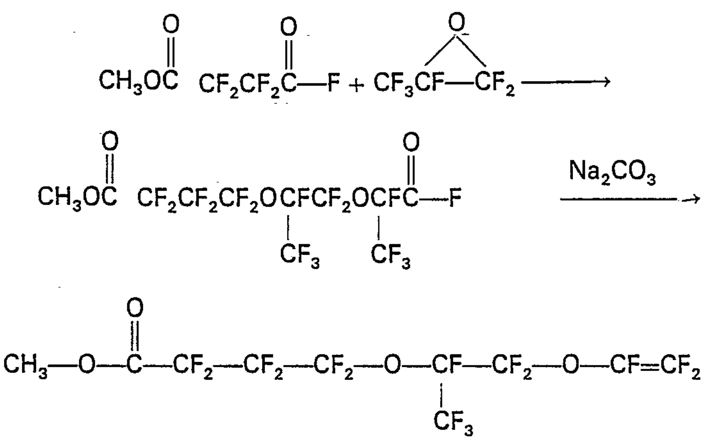


Copolymers of the vinylether monomers with tetrafluoroethylene were shown to be useful as membranes in chlor-alkali electrolytic cells.

Fearn, et al. *Journal of Polymer Science,* Volume 4, pp. 131—140, "Polymers and Terpolymers of Perfluoro-1,4-pentadiene" discloses that in the pyrolysis of sodium salts of carboxylic acids which contain fluorine and chlorine in the $\beta$ position, sodium chloride is preferentially, but not exclusively eliminated. For example

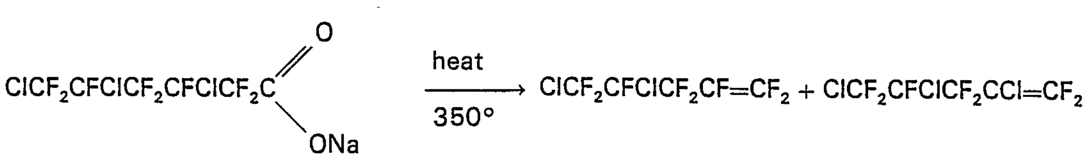


German patent 1,238,458 teaches that useful polymers are made from compounds of the general structure

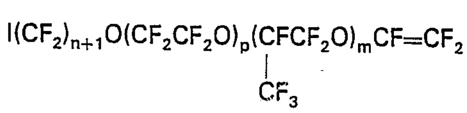

where n = 1—8, p = 0—5 and m = 0—5. Crosslinked halogenated olefin copolymers are produced making use of the iodine group as a reactive site.

R. D. Chambers, in his book, *Fluorine in Organic Chemistry*, published by John Wiley & Sons, 1973, pages 211—212, teaches that carboxylic acid derivatives may be converted to olefins. The conversion is taught to involve the loss of carbon dioxide and formation of an intermediate carbanion. The intermediate then looses NaF to form the resulting olefin.

Numerous other patents and publications have taught the use of acid functional fluorocarbon polymers in chlor-alkali electrolytic cells (British Pats. 1,497,748; 1,497,749; 1,518,387 and U.S. Pats. 3,784,399; 3,969,285; 4,025,405).

Subject of the present invention are novel compounds represented by the formula

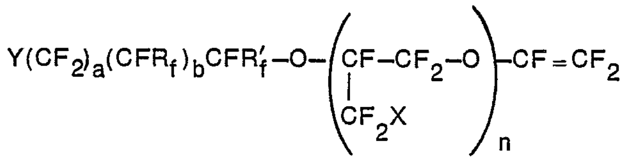

wherein

a is 0 or an integer greater than 0;

b is 0 or an integer greater than 0;

n is one or an integer greater than one;

$R_f'$ and $R_f$ = are independently selected from the group consisting of F, Cl, perfluoroalkyl and fluorochloroalkyl radical;

X = Cl, Br or mixtures thereof when $n > 1$;

Y is selected from the group consisting of $Z'SO_2$,

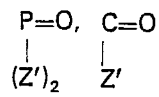

and C ≡ N where Z' is F, Cl, Br, OH, NRR' or OA,

R and R' are independently selected from the group consisting of hydrogen, an alkyl having one or more carbon atoms and an aryl;

A is an alkali metal, quaternary nitrogen radical, or R.

Preferred are the compounds, wherein a = 0—3; b = 0—3; n = 1 to 6; $R_f$ = F or Cl and $R_f'$ = F or Cl, and especially wherein n = 1 and X = Cl. In the method for preparing the compounds preferably X' = Cl and Y = $Z'SO_2$. It is also preferred to carry out the method in the presence of $Na_2CO_3$ as an activator.

Preferably, when $R_f'$, $R_f$, R and R' are alkyl or aryl radicals, they have from 1 to 10, more preferably, from 1 to 4 carbon atoms.

These vinyl ethers may be homopolymerized with themselves or copolymerized with other vinyl ethers.

In the polymerization method preferably a = 0—3; b = 0—3; n = 1—6; $R_f$ = F or Cl and $R_f'$ = F or Cl, and especially n = 1 and X = Cl.

A further subject of the invention is the use of the novel compounds according to the invention in a polymerization reaction, characterized by reacting at least one of these compounds in the presence of an initiator and at a temperature sufficient to cause polymerization together with at least one monomer selected from the group of compounds consisting of tetrafluoroethylene, trifluoromonochloroethylene, trifluoroethylene, vinylidene fluoride, 1,1-difluoro-2,2-dichloroethylene, 1,1-difluoro-2-chloroethylene, hexafluoropropylene, 1,1,1,3,3-pentafluoropropylene, octafluoroisobutylene, ethylene, vinyl chloride, trifluoronitrosomethane, perfluoronitrosoethane, and alkyl vinyl ethers.

Subject of the present invention is therefore also a polymerization method comprising reacting in the presence of an initiator, at least two different types of monomers for a time and a temperature sufficient to cause polymerization:

wherein at least one monomer is selected from a first group of compounds represented by the formula

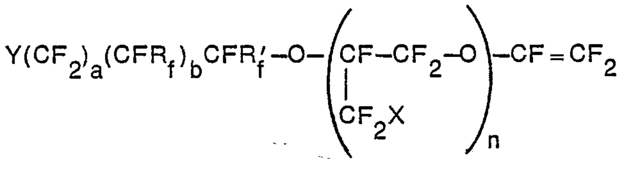

where

n = 1 or an integer greater than 1;

X is Cl, Br or mixtures thereof when $n > 1$;

Y is selected from the group consisting of $Z'SO_2$,

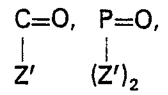

or $C \equiv N$ where Z' is selected from the group consisting of F, Cl, Br, OH, NRR' and OA; where R and R' are independently selected from the group consisting of hydrogen, an alkyl having one or more carbon atoms and an aryl; and A is an alkali metal, a quaternary nitrogen radical, or R;

$R_f$ and $R'_f$ are independently selected from the group consisting of F, Cl, a perfluoroalkyl radical and a chlorofluoroalkyl radical;

a = 0 or an integer greater than 0;

b = 0 or an integer greater than 0; and at least one monomer is selected from a second group of compounds consisting of tetrafluoroethylene, trifluoromonochloroethylene, trifluoroethylene, vinylidene fluoride, 1,1-difluoro-2,2-dichloroethylene, 1,1-difluoro-2-chloroethylene, hexafluoropropylene, 1,1,1,3,3-pentafluoropropylene octafluoroisobutylene, ethylene, vinyl chloride, trifluoronitrosomethane, perfluoronitrosoethane, and alkyl vinyl ethers, and a polymeric composition having pendant groups comprising

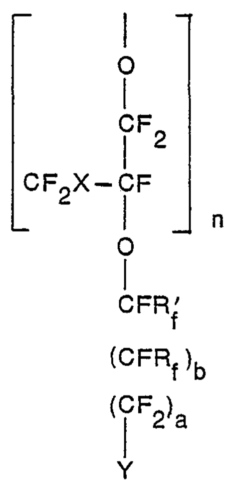

wherein n, X, Y, $R_f$, $R'_f$ a and b have the meanings given above.

A further subject of the invention is a new method of producing the compounds represented by the formula

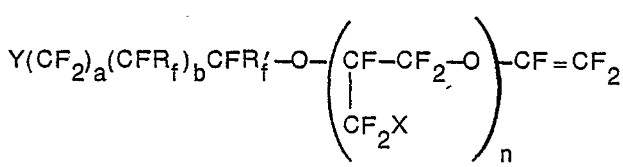

wherein the meanings are as stated above with the proviso, that X can also be F, which comprises decarboxylating compounds of the formula:

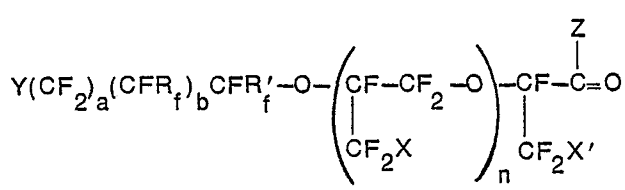

at a temperature and for a time sufficient to form said compound;

wherein

X' = Cl or Br;

Z is F, Cl, Br, OH, NRR' or OA;

and the other meanings are the same as in the end product.

A variety of conditions usually involving a base are generally used for the decarboxylation reaction. Direct reaction of the above compound where Z = F with sodium carbonate as a slurry in a solvent such as glyme, diglyme or tetraglyme is particularly simple. Other methods are pyrolysis of the compound where Z = OH or ONa and reaction of the Z = F compounds with hot $K_2SO_4$ or $Na_2SO_4$ or pyrolysis with ZnO or Silica. Water alone converts the compound directly to the carboxylic acid which can be pyrolyzed. It is generally accepted that conversion of carboxylic acids or derivatives to olefins involves loss of carbon dioxide to form an intermediate carbanion. In the present case producing the following reactive intermediate

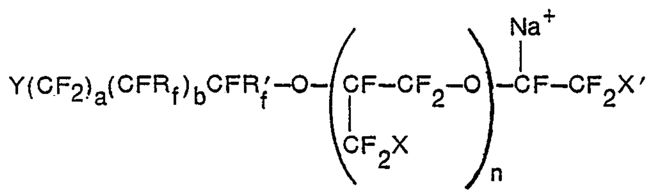

This reactive intermediate then loses NaX′ to form the resulting olefin (monomer). At this point it is also possible to lose NaF which would result in formation of a X′(Cl, Br) substituted olefin, i.e.,

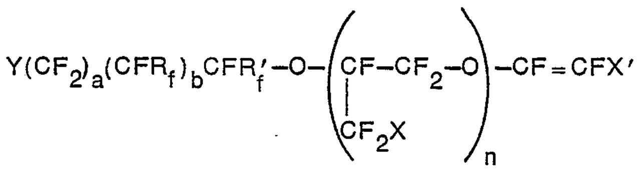

While it is not particularly surprising that loss of NaX′ predominates, it is surprising that loss of NaX′ as opposed to NaF is the sole detected course of the reaction, particularly when X′ = Cl. Loss of NaF, while not being favored over loss of NaX′, does readily occur from similar carbanion intermediates (U.S. 3,282,875). Indeed, Fearn discloses that in the decarboxylation of the structure shown below, elimination of NaCl predominates, but is not exclusive.

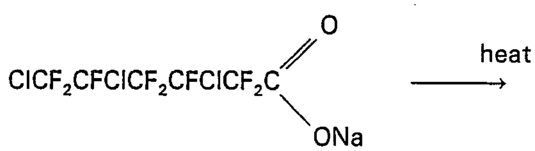


$ClCF_2CFClCF_2CF{=}CF_2$

+

$ClCF_2CFClCF_2CCl{=}CF_2$

Analysis of the vinyl ethers produced by the present invention by VPC, I.R., mass spectroscopy and F 19 NMR failed to detect the presence of any $\sim OCF{=}CFCl$.

The above discussion describes a theory as to how the reactions proceed but in no way limits nor defines the scope of the invention.

When polymers made from the vinyl ether monomers of the present invention are to be formed into sheets for use as membranes, such as in chlor-alkali cells, it is desirable to choose Z so that the polymers formed are thermoplastic to allow fabrication by conventional means, such as melt extrusion, but after fabrication can be easily converted to the acid or alkali metal salt of the acid. As an example, when Y = $SO_2F$ (Z = F), the intermediate is converted to an olefin monomer still having the —$SO_2F$ groups. The monomer is in turn copolymerized to form a polymer containing the $SO_2F$ group that can be formed into sheets by various plastic fabrication techniques. After fabrication, the $SO_2F$ group is easily converted to the alkali metal salt of the corresponding sulfonic acid, —$SO_2ONa$ (Z = ONa), which can be converted to the sulfonic acid, —$SO_2OH$ (Z = OH), by reaction with acids, such as mineral acids.

$$—SO_2F + NaOH \rightarrow —SO_2ONa + NaF \xrightarrow{HCl} —SO_2OH + NaCl$$

When Y is chosen as —C≡N, a nitrile, the above conditions are met since it is well known that nitriles are converted to carboxylic acids by hydrolysis.

When the polymers derived from the present monomer intermediates are to be used in particle or powder form, such as for acid catalyst, it is not critical in the choice of Z since fabrication is not as large

a factor. In this case, Z can conveniently be any of the radicals listed. It can be —OH so as to directly have Y as an acid group or it can be any group rendering Y convertible to an acid group by further reaction, as listed above.

In the decarboxylation reaction the radical X is chosen from the halogens F, Cl or Br, while X′ is chosen from Cl or Br. While iodine would also be a useful radical for X or X′, formation of the ethers by the chemistry taught herein is hampered by side reactions causing low or nonexistant yields to the desired compounds.

When X′ = Cl or Br and X = F, Cl or Br, new uses and novel and surprising new chemistry results from using the intermediates for additional chemical reactions. The prior art teaches that when $Y = SO_2F$, n = 0 and X′ = F (U.S. Patent No. 3,560,568) reaction of the intermediate with base does not produce the desired vinyl ether monomer, but rather a cyclic sulfone compound. Surprisingly, when n = 0, $Y = SO_2F$ and X′ = Cl or Br, reaction of the intermediate with base produces the desired vinyl ether product in one step. In addition to this benefit, choosing X′ = Cl or Br and X = Cl or Br in compounds when n > 0 results in introducing a potential reaction site into polymers ultimately derived from monomers made from these intermediates. When n > 0 both an acid site for ion exchange or catalyst and a reaction site for further reaction can be obtained by having X = Cl or Br and making a copolymer or homopolymer of the vinyl ether. It is known that fluorocarbons having Cl or Br groups undergo metallation reactions to produce reactive intermediates. On the other hand, it is known that these substituents, particularly Cl, do not readily enter into reaction with nucleophiles. Thus the products would be unaffected in normal uses.

There is distinct benefit for having X′ = Cl or Br. It is helpful to have Cl or Br in this position for the decarboxylation reaction. In decarboxylations of the prior art, compounds of the terminal functionality shown below are common.

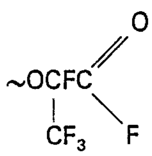

These materials generally require high temperatures and activators such as ZnO or silica to achieve reasonable yields to desired vinyl ethers.

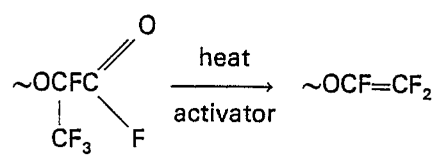


When X′ = Cl or Br in the present invention, decarboxylation of these intermediates to vinyl ethers has been found to proceed under mild conditions and in excellent yields.

The variables have the following preferred values: n = 1—6, a = 0—3 and b = 0—3. Even more preferred are compounds in which n = 1—3. X is preferably Cl and X′ is preferably Cl. $R_f$ and $R'_f$ are preferably F. Y is preferably $Z'SO_2$ and even more preferably $Y = Z'SO_2$ and Z′ = F.

In general, the polymerization procedures and techniques followed in the present invention are known. A very good reference for polymerization techniques is *Emulsion Polymerization — Theory and Practice,* by D. C. Blackley, published by John Wiley & Sons.

Additionally, the copolymers used in the present invention may be prepared by general polymerization techniques developed for homo- and copolymerizations of fluorinated ethylenes, particularly those employed for tetrafluoroethylene which are described in the literature. Nonaqueous techniques for preparing the copolymers of the present invention include that of U.S. Patent No. 3,041,317, to H. H. Gibbs, et al, that is by the polymerization of a mixture of the major monomer therein, such as tetrafluoroethylene, and a fluorinated ethylene containing sulfonyl fluoride in the presence of a free radical initiator, preferably a perfluorocarbon peroxide or azo compound, at a temperature in the range 0°—200°C and at pressures in the range of 1 to 200 atmospheres. The nonaqueous polymerization may, if desired, be carried out in the presence of a fluorinated solvent. Suitable fluorinated solvents are inert, liquid, perfluorinated hydrocarbons, such as perfluoromethylcyclohexane, perfluorodimethylcyclobutane, perfluorooctane, perfluorobenzene and the like.

Aqueous techniques which may also be used for preparing the copolymers used in this invention include contacting the monomers with an aqueous medium containing a free-radical initiator to obtain a slurry of polymer particles in non-waterwet or granular form, as disclosed in U.S. Patent No. 2,393,967 to Brubaker or contacting the monomers with an aqueous medium containing both a free-radical initiator and a technologically inactive dispersing agent, to obtain an aqueous colloidal dispersion of polymer particles and coagulating the dispersion, as disclosed, for example, in U.S. Patent No. 2,559,752 to Berry and U.S. Patent No. 2,593,583 to Lontz.

Any one monomer represented by the general formula may be homopolymerized with itself or any

one monomer may be copolymerized with any other monomer represented by the general formula. Additionally, more than two kinds of monomers represented by the general formula may be polymerized.

In addition, any one or more of the monomers represented by the general formula may be copolymerized with any one or more of the monomers selected from tetrafluoroethylene, trifluoromonochlorethylene, trifluoroethylene, vinylidene fluoride, 1,1-difluoro-2,2-dichloroethylene, 1,1-difluoro-2-chloroethylene, hexafluoropropylene, 1,1,1,3,3-pentafluoropropylene, octafluoroisobutylene ethylene, vinyl chloride, trifluoronitrosomethane, perfluoronitrosoethane or alkyl vinyl ether.

Example 1

50 ml of dry tetraglyme and 8.0 grams anhydrous $Na_2CO_3$ were added to a 100 ml three-neck flask equipped with a stirrer, reflux condenser, thermometer, and a dropping funnel. Cold traps were located downstream of the reflux condenser. 35.67 grams of an acid fluoride product was analyzed and found to contain:

| Percent (wt.) | Compound |
|---|---|
| 12.8 | 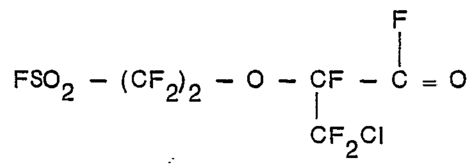 |
| 57.4 | 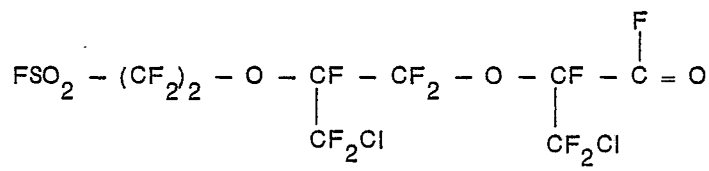 |
| 6.82 | 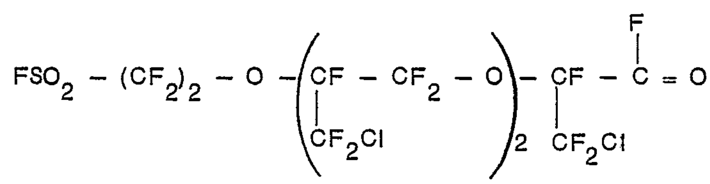 |

It was then added dropwise over a three-hour period to the flask. The temperature rose slightly from room temperature to about 35°C. After $CO_2$ evolution ceased, a 30 inch (1,016.10$^5$ Pa) vacuum was pulled on the system and the reactor was heated slowly until the pot reached 143°C and the overhead temperature reached 99°C. 25.99 grams of product were collected. VPC analysis gave the following results:

| Percent (wt.) | Compound |
|---|---|
| 17.4 | $FSO_2-(CF_2)_2-O-CF=CF_2$ |
| 62.6 | 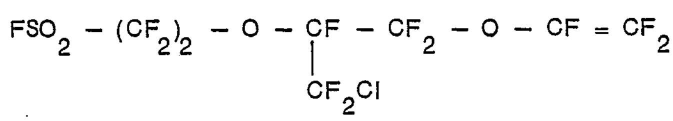 |
| 2.4 | 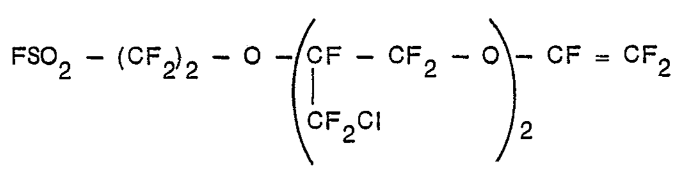 |

Example 2

A 28 gram sample having the following mixture of acid fluorides

| Parts (wt.) | Compound |
|---|---|
| 1 | 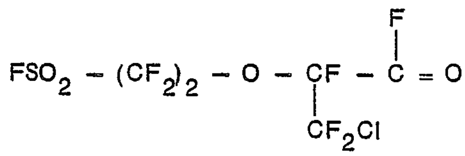 |
| 26 | 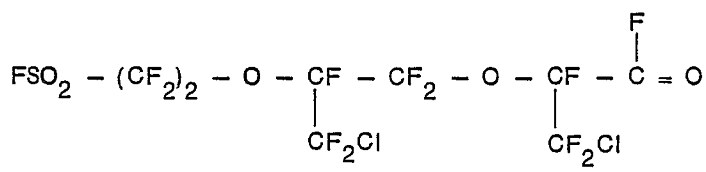 |
| 2.5 | 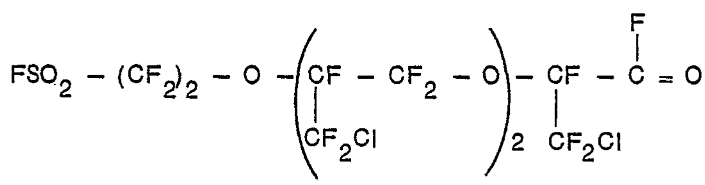 |
| 2 | 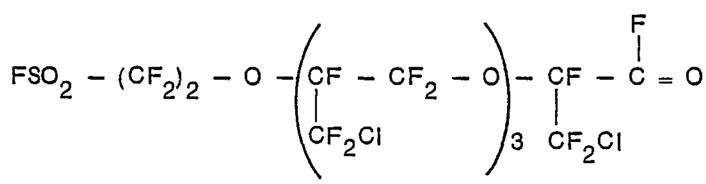 |

was added dropwise to a slurry of 100 ml of freshly distilled tetraglyme and 5 grams $Na_2CO_3$ at 25°C. The mixture was stirred for one hour and then heat was applied slowly. At approximately 80°C, evolution of gas was observed. The products were collected by means of an ordinary Claisen still head with a side arm condenser and a receiver packed in dry ice on the side arm. A nitrogen purge was applied to exclude moisture initially. After gas evolution slowed at 95°C, the receiver was exchanged, a 28 inch Hg ($0{,}948.10^5$ Pa) vacuum was applied and the temperature was raised to 135°C for one hour. Collection was stopped and the two fractions analyzed.

Fraction one had 13.75 grams analyzing as

| Parts (wt.) | Compound |
|---|---|
| 1.7 | $FSO_2-(CF_2)_2-O-CF=CF_2$ |
| 21.7 | 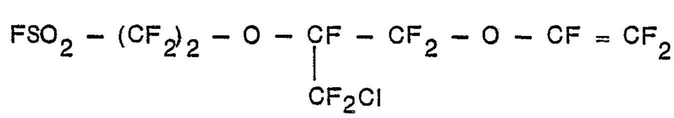 |
| 1 | 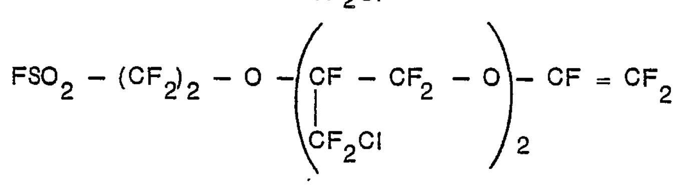 |

Fraction two had 4 grams which was analyzed to be of the following composition:

| Parts (wt.) | Compounds |
|---|---|
| 3.8 | 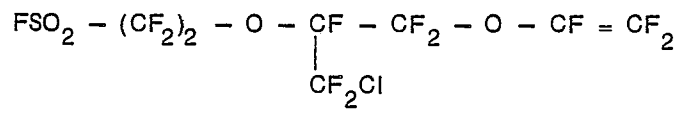 |
| 1 | 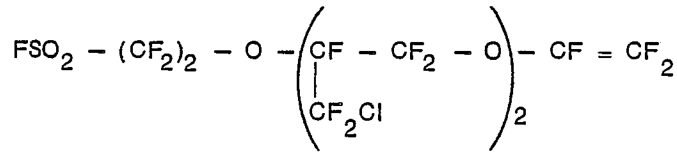 |
| 1 | 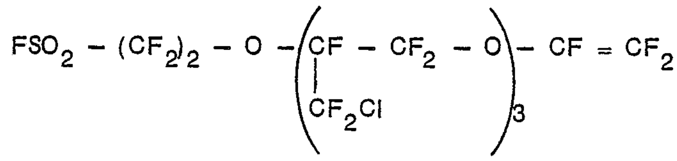 |

Example 3

15 ml of tetraglyme and 1.0 gm of anhydrous $Na_2CO_3$ were added to a 3 neck flask equipped with a thermometer, stirrer and reflux condenser. Cold traps (−78°C) were downstream of the condenser and a slight backpressure of $N_2$ was maintained by means of a bubbler.

$FSO_2CF_2CF_2OCF(CF_3)CF_2OCF(CF_2Cl)CFO$ (3 gms)

was added and after a brief evolution of $CO_2$, the temperature was raised to 80°C and held there for several hours until $CO_2$ evolution ceased. A vacuum was pulled on the reactor and the temperature was slowly increased to 136°C while collecting 1.5 gms of product in the cold trap. The majority of the product was collected before the temperature reached 90°C. VPC analysis showed additional product remaining in the tetraglyme solvent. The product was confirmed as

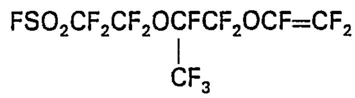

by Mass Spectroscopy, I.R. (Infra Red) and $F^{19}$ NMR.

Using the procedures of the foregoing examples, the following functional fluorovinyl ethers are prepared:

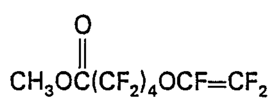

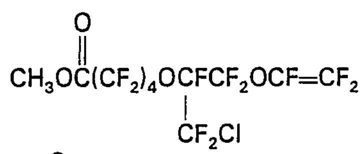

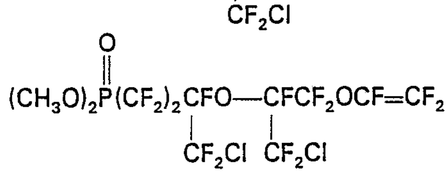

Example 4

$FSO_2-(CF_2)_2-O-CF=CF_2$, 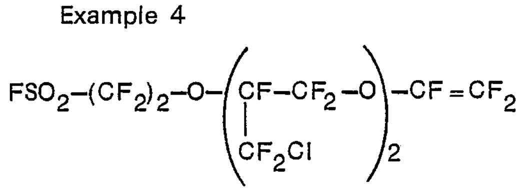

and tetrafluoroethylene were polymerized together as follows:

Ten grams of a mixture of

$FSO_2-(CF_2)_2-O-CF=CF_2$ and 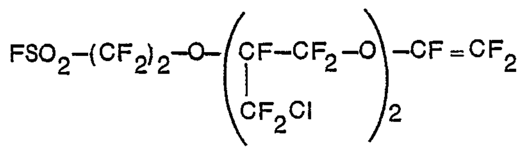

were added to 400 ml of a deoxygenated water solution containing 3 gm $K_2S_2O_8$, .75 gm $NaHSO_3$, 1.5 gm $Na_2HPO_4$, and 3.5 gm $C_7F_{15}CO_2K$ of a surfactant in a glass-lined autoclave. The reaction was then carried out by maintaining a 60 psi ($2,72.10^4 g/6.45\ cm^2$) tetrafluoroethylene pressure on the reactor for 16 hours with stirring at 10°C. The reactor was then vented, heated to 50°C and evacuated to remove residual monomer. The contents were then frozen to coagulate the polymer which was filtered and vigorously washed after thawing. The dried polymer weighed 27.5 gm. A film pressed from the polymer exhibited bands in the infrared absorption region associated with the $—SO_2F$ group at 820 and 1465 cm˙1.

Example 5

10 gm of a mixture containing approximately two parts

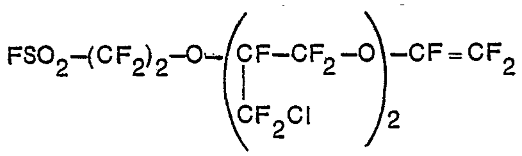 and one part

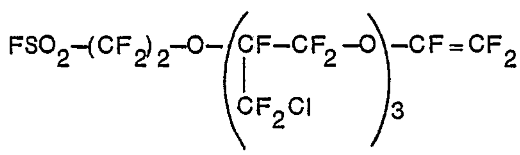

was added to a glass-lined, stirred stainless steel reactor containing 3 gm $K_2S_2O_8$, 0.75 gm $NaHSO_3$, 1.5 gm $Na_2HPO_4$, and 3.5 gm $C_7F_{15}CO_2K$. A 60 psi pressure of tetrafluoroethylene was then applied to the reactor and the temperature maintained at 20°C for 1 3/4 hours. The reactor was vented, vacuum applied and heated to 50°C to remove volatiles. The contents were then frozen, thawed and filtered followed by vigorous washing to remove inorganics and surfactant. The vacuum dried polymer weighed 7 gm. Titration of a sample of the polymer, after hydrolysis from the $—SO_2—F$ to the $SO_2—ONa$ form using NaOH in an ethanol and water mixture, gave a value of 3409 for the equivalent weight.

Example 6

9.25 gm of

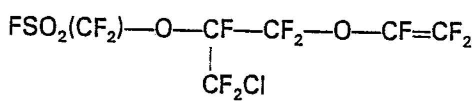

were added to 30 ml of $ClCF_2—CCl_2F$ in a 100 ml stainless steel reactor. The contents were cooled to the freezing point and 2 drops of 2-tert. butylazo-2-cyano-4-methoxyl-4-methylpentane initiator solution added. The reactor was then evaporated and 8 gm of tetrafluoroethylene added by condensation. The reactor was heated to 50°C and shaken for 14 hours. The reactor was then vented and the solvent evaporated leaving a dried polymer residue of 4 gm. The polymer was analyzed as containing 0.8% sulfur.

Example 7

4.5 gm of

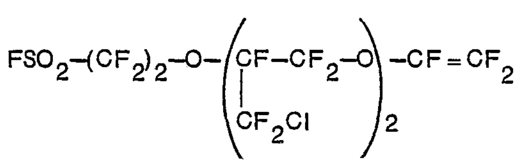

were added to 30 ml of $ClCF_2—CFCl_2$in a 100 ml stainless steel reactor. The contents were cooled to the freezing point, 2 drops of 2-tert. butylazo-2-cyano-4-methoxyl-4-methylpentane initiator solution were added. The reactor was then evacuated and 8.25 gm tetrafluoroethylene added by condensation. The reactor was then heated to 50°C and shaken for 22 hours. The reactor was vented and the solvent evaporated, leaving a dried polymer residue of 7 gm which analyzed as containing 0.6% sulfur.

**Claims**

1. As a composition of matter, compounds represented by the formula

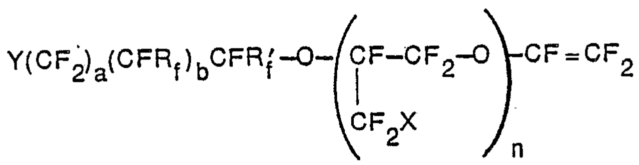

where
$n = 1$ or an integer greater than 1;
$X = Cl$, Br or mixtures thereof when $n > 1$;
Y is selected from the group consisting of $Z'SO_2$,

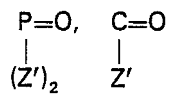

and C≡N where Z′ is F, Cl, Br, OH, NRR′ or OA, R and R′ are independently selected from the group consisting of hydrogen, an alkyl having one or more carbon atoms and an aryl,
A is an alkali metal, quaternary nitrogen radical, or R;
$R_f$ and $R'_f$ are independently selected from the group consisting of F, Cl, a perfluoroalkyl radical and a chlorofluoroalkyl radical;
a=0 or an integer greater than 0;
b=0 or an integer greater than 0.

2. The compounds of Claim 1 wherein $a = 0$—3; $b = 0$—3; $n = 1$ to 6; $R_f = F$ or Cl and $R'_f = F$ or Cl.

3. The compounds of Claim 1 or 2 wherein $n = 1$ and $X = Cl$.

4. A method of producing the compounds represented by the formula

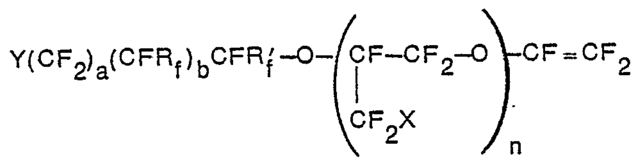

where
$n = 1$ or an integer greater than 1;
$X = F$, Cl, Br or mixtures thereof when $n > 1$;
Y is selected from the group consisting of $Z'SO_2$,

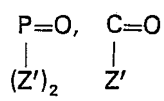

and C≡N where Z′ is F, Cl, Br, OH, NRR′ or OA, R and R′ are independently selected from the group consisting of hydrogen, an alkyl having one or more carbon atoms and an aryl,
A is an alkali metal, quaternary nitrogen radical, or R;
$R_f$ and $R'_f$ are independently selected from the group consisting of F, Cl, a perfluoroalkyl radical and a chlorofluoroalkyl radical;
a=0 or an integer greater than 0;
b=0 or an integer greater than 0,
which comprises decarboxylating compounds of the formula:

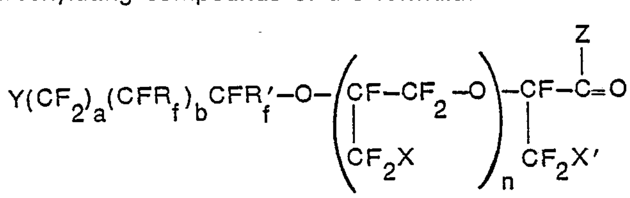

at a temperature and for a time sufficient to form said compound;
wherein
$X' = Cl$ or Br;
Z is F, Cl, Br, OH, NRR′ or OA;
and the other meanings are the same as in the end product.

5. The method of Claim 4 wherein $a = 0$—3; $b = 0$—3; $R_f = F$ or Cl; $R'_f = F$ or Cl and $n = 1$—6.

6. The method of Claim 4 or 5 wherein n = 1; X′ = Cl and Y = $Z'SO_2$.

7. The method of any one of Claims 4 to 6 wherein n = 1; X = Cl and X′ = Cl.

8. The method of Claim 4 wherein the method is carried out in the presence of $Na_2CO_3$ as an activator.

9. A polymerization method comprising reacting in the presence of an initiator, at least two different types of monomers for a time and a temperature sufficient to cause polymerization:

wherein at least one monomer is selected from a first group of compounds represented by the formula

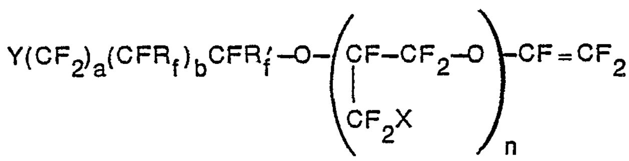

where

n = 1 or an integer greater than 1;

X is Cl, Br or mixtures thereof when n > 1;

Y is selected from the group consisting of $Z'SO_2$,

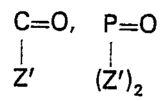

or C≡N where Z′ is selected from the group consisting of F, Cl, Br, OH, NRR′ or OA; where R and R′ are independently selected from the group consisting of hydrogen, an alkyl having one or more carbon atoms and an aryl, and A is an alkali metal, a quaternary nitrogen radical, or R;

$R_f$ and $R'_f$ are independently selected from the group consisting of F, Cl, a perfluoroalkyl radical and a chlorofluoroalkyl radical;

a=0 or an integer greater than 0;

b=0 or an integer greater than 0; and at least one monomer is selected from a second group of compounds consisting of tetrafluoroethylene, trifluoromonochloroethylene, trifluoroethylene, vinylidine fluoride, 1,1,-difluoro-2,2-dichloroethylene, 1,1-difluoro-2-chloroethylene, hexafluoropropylene, 1,1,1,3,3-pentafluoropropylene, octafluoroisobutylene, ethylene, vinyl chloride, trifluoronitrosomethane, perfluoronitrosoethane, and alkyl vinyl ethers.

10. The method of Claim 9 wherein a = 0—3; b = 0—3; n = 1—6; $R_f$ = F or Cl and $R'_f$ = F or Cl.

11. The method of Claim 9 or 10, wherein n = 1 and X = Cl.

12. A polymeric composition having pendant groups comprising

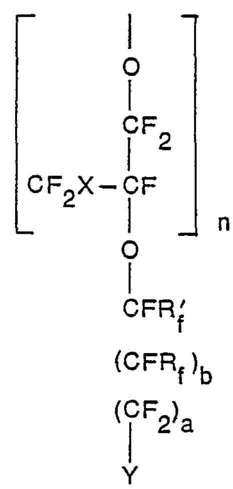

where

n = 1 or an integer greater than 1;

X = Cl, Br or mixtures thereof when n > 1;

Y is selected from the group consisting of $Z'SO_2$,



and C≡N where Z′ is F, Cl, Br, OH, NRR′ or OA,

R and R′ are independently selected from the group consisting of hydrogen, an alkyl having one or more carbon atoms and an aryl,

A is an alkali metal, quaternary nitrogen radical, or R;

$R_f$ and $R_f'$ are independently selected from the group consisting of F, Cl, a perfluoroalkyl radical and a chlorofluoroalkyl radical;

a=0 or an integer greater than 0;

b=0 or an integer greater than 0.

13. Use of a compound according to any of Claims 1 to 3 in a polymerization reaction, characterized by reacting at least one of these compounds in the presence of an initiator and at a temperature sufficient to cause polymerization together with at least one monomer selected from the group of compounds consisting of tetrafluoroethylene, trifluoromonochloroethylene, trifluoroethylene, vinylidene fluoride, 1,1-difluoro-2,2-dichloroethylene, 1,1-difluoro-2-chloroethylene, hexafluoropropylene, 1,1,1,3,3-pentafluoropropylene, octafluoroisobutylene, ethylene, vinyl chloride, trifluoronitrosomethane, perfluoronitrosoethane, and alkyl vinyl ethers.

**Revendications**

1. Composés représentés par la formule:

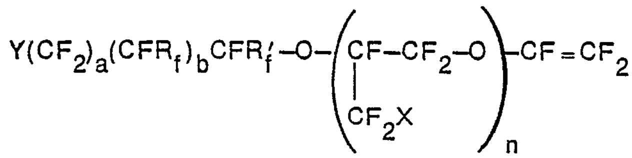

dans laquelle:

—$n = 1$ ou un nombre entier supérieur à 1;

—X = Cl, Br ou leurs mélanges lorsque $n > 1$;

—Y est choisi dans le groupe formé par: $Z'SO_2$,

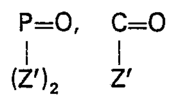

et C≡N,

dans lesquels Z′ est F, Cl, Br, OH, NRR′ ou OA, R et R′ étant indépendamment choisis dans le groupe formé par l'hydrogène, un alkyle ayant un ou plusieurs atomes de carbone et les radicaux aryle, et A étant un métal alcalin, un radical azote quaternaire, ou R;

—$R_f$ et $R_f'$ sont indépendamment choisis dans le groupe formé par F, Cl, les radicaux perfluoroalkyl et les radicaux chlorofluoroalkyle;

—$a = 0$ ou un nombre entier supérieur à 0;

—$b = 0$ ou un nombre entier supérieur à 0.

2. Composés selon la revendication 1 dans lesquels $a = 0$—3; $b = 0$—3; $n = 1$ à 6; $R_f$ =F ou Cl et $R_f'$ = F ou Cl.

3. Composés selon la revendication 1 ou 2 lesquels $n = 1$ et X = Cl.

4. Procédé de préparation des composés représentés par la formule:

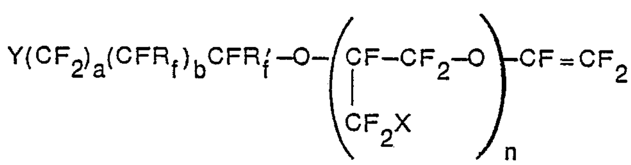

dans laquelle:

—$n = 1$ ou un nombre entier supérieur à 1;

—X = F, Cl, Br ou leurs mélanges lorsque $n > 1$;

—Y est choisi dans le groupe formé par: $Z'SO_2$,

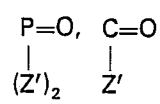

et C≡N,

dans lesquels Z′ est F, Cl, Br, OH, NRR′ ou OA, R et R′ étant indépendamment choisis dans le groupe formé par l'hydrogène, un alkyle ayant un ou plusieurs atomes de carbone et les radicaux aryle, et A étant un métal alcalin, un radical azote quaternaire, ou R;

—$R_f$ et $R'_f$ sont indépendamment choisis dans le groupe formé par F, Cl, les radicaux perfluoroalkyle et les radicaux chlorofluoroalkyle;

—a = 0 ou un nombre entier supérieur à 0;

—b = 0 ou un nombre entier supérieur à 0,

consistant à décarboxyler, à une température et pendant un temps suffisants pour former lesdits composés, des composés de formule:

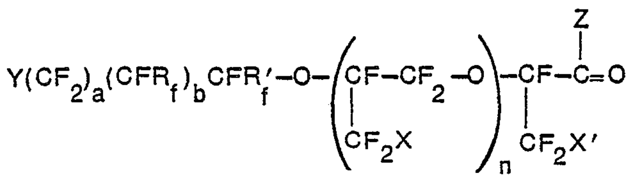

dans laquelle:

—X' = Cl ou Br;

—Z est F, Cl, Br, OH, NRR' ou OA;

les autres symboles ayant les mêmes que ceux du produit final.

5. Procédé selon la revendication 4, dans lequel a = 0—3; b = 0—3; $R_f$ = F ou Cl; $R'_f$ = F ou Cl et n = 1—6.

6. Procédé selon la revendication 4 ou 5, dans lequel n = 1; X' = Cl et Y = $Z'SO_2$.

7. Procédé selon l'une des revendications 4 à 6, dans lequel n = 1; X = Cl et X' = Cl.

8. Procédé selon la revendication 4, dans lequel on met en oeuvre ledit procédé en présence de $Na_2CO_3$ comme activateur.

9. Procédé de polymérisation consistant à faire réagir en présence d'un initiateur, au moins deux types différents de monomères pendant un temps et une température suffisants pour provoquer une polymérisation:

dans lequel au moins un monomère est choisi dans un premier groupe de composés représentés par la formule:

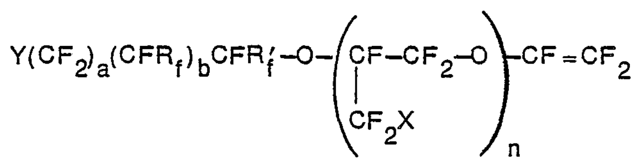

dans laquelle:

—n = 1 ou un nombre entier supérieur à 1;

—X = Cl, Br ou leurs mélanges lorsque n > 1;

—Y est choisi dans le groupe formé par: $Z'SO_2$,

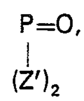,
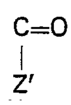

et C≡N,

dans lesquels Z' est F, Cl, Br, OH, NRR' ou OA, R et R' étant indépendamment choisis dans le groupe formé par l'hydrogène, un alkyle ayant un ou plusieurs atomes de carbone et les radicaux aryle, et A étant un métal alcalin, un radical azote quaternaire, ou R;

—$R_f$ et $R'_f$ sont indépendamment choisis dans le groupe formé par F, Cl, les radicaux perfluoroalkyle et les radicaux chlorofluoroalkyle;

—a = 0 ou un nombre entier supérieur à 0;

—b = 0 ou un nombre entier supérieur à 0.

et au moins un monomère est choisi dans un second groupe de composés formé par le tétrafluoroéthylène, le trifluoromonochloroéthylène, le trifluoroéthylene, le fluorure de vinylidène, le 1,1-difluoro-2,2-dichloroéthylène, le 1,1-difluoro-2-chloroéthylène, l'hexafluoropropylène, le1,1,1,3,3-pentafluoropropylène, l'octafluoroisobutylène, l'éthylène, le chlorure de vinyle, le trifluoronitrosométhane, le perfluoronitrosoéthane, et les alkyl vinyl éthers.

10. Procédé selon la revendication 9, dans lequel a = 0—3; b = 0—3; n = 1—6; $R_f$ = F ou Cl et $R'_f$ = F ou Cl.

11. Procédé selon la revendication 9 ou 10, dans lequel n = 1 et X = Cl.

12. Composition polymère ayant des groupes pendants correspondant à la formule suivante:

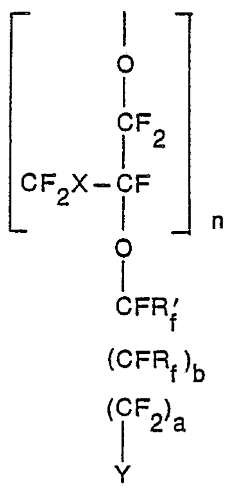

dans laquelle:

—n = 1 ou un nombre entier supérieur à 1;

—X = Cl, Br ou leurs mélanges lorsque n > 1;

—Y est choisi dans le groupe formé par: $Z'SO_2$,

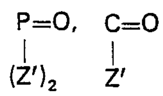

et C≡N,

dans lesquels Z′ est F, Cl, Br, OH, NRR′ ou OA, R et R′ étant indépendamment choisis dans le groupe formé par l'hydrogène, un alkyle ayant un ou plusieurs atomes de carbone et les radicaux aryle, et A étant un métal alcalin, un radical azote quaternaire, ou R;

—$R_f$ et $R'_f$ sont indépendamment choisis dans le groupe formé par F, Cl, les radicaux perfluoroalkyle et les radicaux chlorofluoroalkyle;

—a = 0 ou un nombre entier supérieur à 0;

—b = 0 ou un nombre entier supérieur à 0.

13. Utilisation d'un composé selon l'une des revendications 1 à 3, dans une réaction de polymérisation, caractérisée par le fait que l'on fait réagir au moins l'un de ces composés en présence d'un initiateur et à une température suffisante pour provoquer leur polymérisation conjointement avec au moins un monomèrie choisi dans le groupe des composés constitué par le térafluoroéthylène, le trifluoromonochloroéthylène, le trifluoroéthylène, le fluorure de vinylidène, le 1,1-difluoro-2,2-dichloroéthylène, le 1,1-difluoro-2-chloroéthylène, l'hexafluoropropylène, le 1,1,1,3,3-pentafluoropropylène, l'octafluoroisobutylène, l'éthylène, le chlorure de vinyle, le trifluoronitrosométhane, le perfluoronitrosoéthane, et les alkyl vinyl éthers.

**Patentansprüche**

1. Verbindungen der Formel

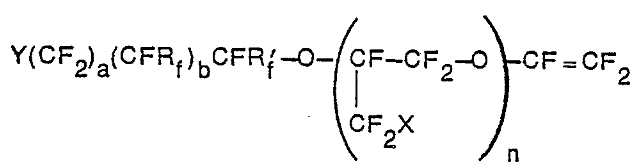

worin

n = 1 oder eine ganze Zahl größer als 1;

X = Chlor, Brom oder Mischungen davon, wenn n > 1;

Y ausgewählt ist aus der Gruppe bestehend aus $Z'SO_2$,

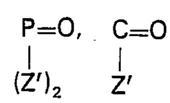

und C≡N, worin Z′ Fluor, Chlor, Brom, OH, NRR′ oder OA ist,

R und R′ unabhängig voneinander ausgewählt sind aus der Gruppe bestehend aus Wasserstoff, einem Alkyl mit einem oder mehreren Kohlenstoffatomen und einem Aryl,

A ein Alkalimetall, quaternäres Stickstoffradikal ist, oder R;

$R_f$ und $R_f'$ unabhängig voneinander ausgewählt sind aus der Gruppe bestehend aus F, Cl, einem Perfluoroalkylradikal und einem Chlorfluoralkylradikal;

a = 0 oder eine ganze Zahl größer als 0;

b = 0 oder eine ganze Zahl größer als 0.

2. Verbindungen nach Anspruch 1, dadurch gekennzeichnet, daß a = 0—3; b = 0—3; n = 1—6; $R_f$ = F oder Cl und $R_f'$ = F oder Cl.

3. Verbindungen nach Anspruch 1 oder 2, dadurch gekennzeichnet, daß n = 1 und X = Cl.

4. Verfahren zur Herstellung der Verbindungen der Formel

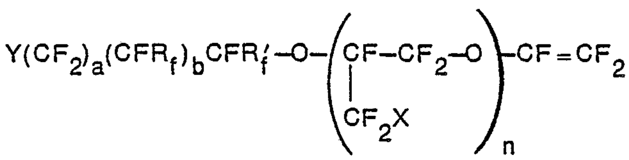

worin

n = 1 oder eine ganze Zahl größer als 1;

X = F, Cl, Br oder Mischungen davon, wenn n > 1;

Y ausgewählt ist aus der Gruppe bestehend aus $Z'SO_2$,

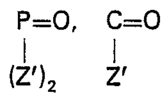

und C≡N, worin Z' F, Cl, Br, OH, NRR' oder OA ist,

R und R' unabhängig voneinander ausgewählt sind aus der Gruppe bestehend aus Wasserstoff, einem Alkyl mit einem oder mehreren Kohlenstoffatomen und einem Aryl,

A ein Alkalimetall, quaternäres Stickstoffradikal oder R ist;

$R_f$ und $R_f'$ unabhängig voneinander ausgewählt sind aus der Gruppe bestehend aus F, Cl, einem Perfluoroalkylradikal und einem Chlorfluoralkylradikal;

a = 0 oder eine ganze Zahl größer als 0;

b = 0 oder eine ganze Zahl größer als 0,

dadurch gekennzeichnet, daß man Verbindungen der Formel

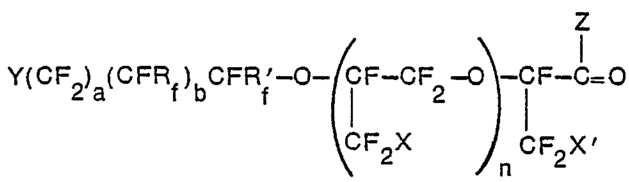

worin

X' = Cl oder Br;

Z = F, Cl, Br, OH, NRR' oder OA;

und die anderen Bedeutungen die gleichen wie im Endprodukt sind,

bei einer Temperatur und einer Zeit, die zur Bildung der genannten Verbindung ausreichend ist, decarboxyliert.

5. Verfahren nach Anspruch 4, dadurch gekennzeichnet, daß a = 0—3; b = 0—3; $R_f$ = F oder Cl; $R_f'$ = F oder Cl und n = 1—6.

6. Verfahren nach Anspruch 4 oder 5, dadurch gekennzeichnet, daß n = 1; X' = Cl und Y = $Z'SO_2$.

7. Verfahren nach einem der Ansprüche 4 bis 6, dadurch gekennzeichnet, daß n = 1; X = Cl und X' = Cl.

8. Verfahren nach Anspruch 4, dadurch gekennzeichnet, daß das Verfahren in Gegenwart von $Na_2CO_3$ als Aktivator durchgeführt wird.

9. Polymerisationsverfahren, bei dem in Gegenwart eines Initiators mindestens zwei verschiedene Monomerentypen während einer Zeit und bei einer Temperatur, die zur Verursachung der Polymerisation ausreicht, zur Reaktion gebracht werden, dadurch gekennzeichnet, daß mindestens ein Monomeres ausgewählt ist aus einer ersten Gruppe von Verbindungen der Formel

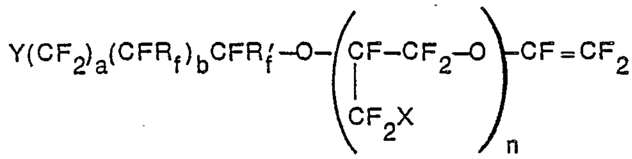

worin

n = 1 oder eine ganze Zahl größer als 1;

X = Cl, Br oder Mischungen davon, wenn n > 1;

Y ausgewählt ist aus der Gruppe bestehend aus $Z'SO_2$,

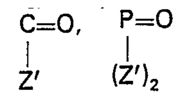

oder C ≡ N, worin Z' ausgewählt ist aus der Gruppe bestehend aus F, Cl, Br, OH, NRR' und OA; worin R und R' unabhängig voneinander ausgewählt sind aus der Gruppe bestehend aus Wasserstoff, einem Alkyl mit einem oder mehreren Kohlenstoffatomen und einem Aryl; und A ein Alkalimetall, ein quaternäres Stickstoffradikal oder R ist;

$R_f$ und $R'_f$ unabhängig voneinander ausgewählt sind aus der Gruppe bestehend aus F, Cl, einem Perfluoralkylradikal und einem Chlorfluoralkylradikal;

a = 0 oder eine ganze Zahl größer als 0;

b = 0 oder eine ganze Zahl größer als 0; und

mindestens ein Monomeres ausgewählt ist aus einer zweiten Gruppe von Verbindungen, bestehend aus Tetrafluoräthylen, Trifluormonochloräthylen, Trifluoräthylen, Vinylidenfluorid, 1,1-Difluor-2,2-dichloräthylen, 1,1-Difluor-2-chloräthylen, Hexafluorpropylen, 1,1,1,3,3-Pentafluorpropylen, Octafluorisobutylen, Äthylen, Vinylchlorid, Trifluornitrosomethan, Perfluornitrosoäthen und Alkylvinyläthern.

10. Verfahren nach Anspruch 9, dadurch gekennzeichnet, daß a = 0—3; b = 0—3; n = 1—6; $R_f$ = F oder Cl und $R'_f$ = F oder Cl.

11. Verfahren nach Anspruch 9 oder 10, dadurch gekennzeichnet, daß n = 1 und X = Cl.

12. Polymerzusammensetzung mit seitlichen (pendant) Gruppen enthaltend

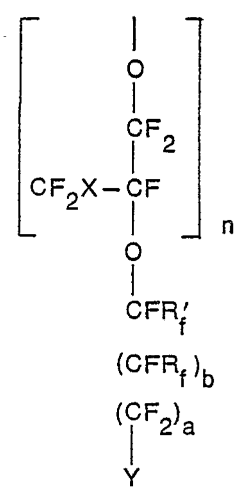

worin

n = 1 oder eine ganze Zahl größer als 1;

X = Cl, Br oder Mischungen davon, wenn n > 1;

Y ausgewählt ist aus der Gruppe bestehend aus $Z'SO_2$,

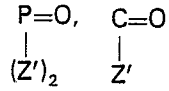

und C≡N, worin Z' F, Cl, Br, OH, NRR' oder OA,

R und R' unabhängig voneinander ausgewählt sind aus der Gruppe bestehend aus Wasserstoff, einem Alkyl mit einem oder mehreren Kohlenstoffatomen und einem Aryl,

A ein Alkalimetall, quaternäres Stickstoffradikal oder R ist;

$R_f$ und $R'_f$ unabhängig voneinander ausgewählt sind aus der Gruppe bestehend aus F, Cl, einem Perfluoroalkylradikal und einem Chlorfluoralkylradikal;

a = 0 oder eine ganze Zahl größer als 0;

b = 0 oder eine ganze Zahl größer als 0.

13. Verwendung einer Verbindung nach einem der Ansprüche 1 bis 3 in einer Polymerisationsreaktion, dadurch gekennzeichnet, daß man mindestens eine dieser Verbindungen in Gegenwart eines Initiators und bei einer Temperatur, die zur Verursachung der Polymerisation ausreicht, reagieren läßt zusammen mit mindestens einem Monomer ausgewählt aus der Gruppe der Verbindungen bestehend aus Tetrafluoräthylen, Trifluormonochloräthylen, Trifluoräthylen, Vinylidenfluorid, 1,1-difluor-2,2-dichloräthylen, 1,1-Difluor-2-chloräthylen, Hexafluorpropylen, 1,1,1,3,3-Pentafluorpropylen, Octafluorisobutylen, Äthylen, Vinylchlorid, Trifluornitrosomethan, Perfluornitrosoäthan und Alkylvinyläthern.